# EUROPÄISCHE PATENTSCHRIFT

(11) **EP 1 715 837 B1**
(45) Veröffentlichungstag und Bekanntmachung des Hinweises auf die Patenterteilung: **25.02.2015**
(21) Anmeldenummer: 04803937.4
(22) Anmeldetag: 16.12.2004
(51) Int. Cl.: A61K 8/37, A61K 8/41, A61Q 5/06, A61Q 5/10, A61Q 5/12

(54) **KATIONISCHE CREMEGRUNDLAGE**
CATIONIC CREAM BASE
BASE DE CREME CATIONIQUE

(30) Priorität: 24.12.2003 DE 10361278; 27.09.2004 DE 102004047137
(43) Veröffentlichungstag der Anmeldung: 02.11.2006
(73) Patentinhaber: Henkel AG & Co. KGaA, 40589 Düsseldorf (DE)
(72) Erfinder: WOLFF, Wolfgang, 22941 Bargteheide (DE); MANNECK, Hartmut, 23860 Klein Wesenberg (DE); WEISHAUPT, Sarah, 20355 Hamburg (DE); AKRAM, Mustafa, 22457 Hamburg (DE); KLEEN, Astrid, 22763 Hamburg (DE); KAWAI, Masaki, Ryugassaki-Shi, Ibaraki-Ken 301-0044 (JP)
(86) Internationale Anmeldenummer: PCT/EP2004/014324
(87) Internationale Veröffentlichungsnummer: WO 2005/063179

(56) Entgegenhaltungen:
- EP-A- 1 048 289
- EP-A- 1 224 927
- EP-A- 1 371 353
- WO-A-00/76467
- WO-A2-01/15662
- DE-A1- 4 234 744
- US-A- 6 156 076
- DATABASE CA [Online] CHEMICAL ABSTRACTS SERVICE, COLUMBUS, OHIO, US; 27. Februar 2004 (2004-02-27), MIURA, HIDEKI ET AL: "Hair dye compositions having hair conditioning effect" XP002328346 gefunden im STN Database accession no. 2004:159354 -& JP 2004 059565 A (SANEI KAGAKU CO., LTD., JAPAN) 26. Februar 2004 (2004-02-26)
- DATABASE GNPD [Online] MINTEL 31 Januar 2003 'Prominous Permanent Colour' Database accession no. 183950
- DATABASE GNPD [Online] MINTEL 31 Oktober 2003 'Creme-Gel Color' Database accession no. 10150469
- DATABASE GNPD [Online] MINTEL 31 August 2002 'Hair Color Cream-Gel' Database accession no. 10116321

## Beschreibung

Die vorliegende Anmeldung betrifft Färbemittel für keratinische Fasern, enthaltend neben Farbstoffvorprodukten mindestens 0,1 bis 10 Gew.-% Fettsäureglycerylester sowie mindestens ein spezielles kationisches Tensid und mindestens ein vollständig oder partiell hydriertes Pflanzenöl.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Für dauerhafte, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Die Oxidationsfärbemittel zeichnen sich durch hervorragende, lang anhaltende Färbeergebnisse aus. Für natürlich wirkende Färbungen muss üblicherweise eine Mischung aus einer größeren Zahl von Oxidationsfarbstoffvorprodukten eingesetzt werden; in vielen Fällen werden weiterhin direktziehende Farbstoffe zur Nuancierung verwendet.

Schließlich hat in jüngster Zeit ein neuartiges Färbeverfahren große Beachtung gefunden. Bei diesem Verfahren werden Vorstufen des natürlichen Haarfarbstoffes Melanin auf das Haar aufbracht; diese bilden dann im Rahmen oxidativer Prozesse im Haar naturanaloge Farbstoffe aus. Ein solches Verfahren mit 5,6-Dihydroxyindolin als Farbstoffvorprodukt wurde in der EP-B1-530 229 beschrieben. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss. Bei Personen mit ursprünglich mittelblondem bis braunem Haar kann das Indolin als alleinige Farbstoffvorstufe eingesetzt werden. Für die Anwendung bei Personen mit ursprünglich roter und insbesondere dunkler bis schwarzer Haarfarbe können dagegen befriedigende Ergebnisse häufig nur durch Mitverwendung weiterer Farbstoffkomponenten, insbesondere spezieller Oxidationsfarbstoffvorprodukte, erzielt werden.

Üblicherweise werden Haarfärbemittel in Form wässriger Emulsionen oder Färbegele formuliert, die gegebenenfalls unmittelbar vor der Anwendung mit einer Oxidationsmittelzubereitung vermischt werden. Bei der Formulierung von Haarfärbemitteln in Form von Emulsionen sind aufgrund des teilweise sehr hohen Salzgehaltes der Rezepturen häufig Probleme bei der Lagerstabilität zu beobachten. Der Salzgehalt kann unter anderem aus den häufig in Form der lagerstabileren Salze eingesetzten Farbstoffvorprodukten sowie Salz-Beimengungen, die aus dem Herstellungsprozess insbesondere bei direktziehenden Farbstoffen stammen, resultieren.

Um den Pflegezustand der Fasern zu verbessern ist es seit langem üblich, die Fasern im Anschluss an die farbverändernde Behandlung einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splissrate verringert.

In jüngster Zeit wurden ferner sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die im Rahmen derartiger Kombinationspräparate einsetzbaren Wirkstoffe müssen insbesondere hinsichtlich ihrer Stabilität hohen Ansprüchen genügen, da die Färbecremes üblicherweise einen hohen pH-Wert und die Oxidationsmittelzubereitungen einen niedrigen pH-Wert aufweisen. Ferner sind Unverträglichkeiten der verschiedenen Wirkstoffe untereinander und somit eine geringe Lagerstabilität zu vermeiden.

Im Rahmen der Anmeldungen DE-A-199 14 927, DE-A-199 14 926 und DE-A-44 08 506 wurden bereits derartige Wirkstoffkombinationen zum Einsatz in oxidativen Färbemitteln vorgeschlagen. Dennoch lassen auch diese Mittel, insbesondere auf schwer zu pflegenden Fasern, wie beispielsweise japanischem Haar, noch einige Wünsche hinsichtlich der pflegenden Eigenschaften offen.

EP 1 048 289 A offenbart Haarfärbepräparate, welche Fettsäure(polyglycol)ester, Fettsäurepartialglyceride und Haarfarbstoffe in jeweils bestimmten Mengenbereichen enthalten. Unter den Namen "Prominous Permanent Colour" (Hoyu), "Creme-Gel Color" (Revlon Professional) und "Hair Color Cream-Gel" (Fermodyl) sind im Handel verschiedene Haarfärbemittel erhältlich, welche Farbstoffvorprodukte, Glycerylstearat und Steartrimoniumchlorid in unbestimmten Mengenbereichen enthalten. WO 01/15662 A2 betrifft zweistufige Verfahren zum Farben von keratinischen Fasern, in denen Formulierungen mit Oxidationsfarbstoffvorprodukten, Glycerylstearat und einem kationsichen Tensid eingesetzt werden.

Es besteht daher weiterhin ein Bedarf an Cremegrundlagen für die farbverändernde Behandlung von Fasern mit guten pflegenden Eigenschaften und einer guten Stabilität auch bei Formulierungen mit einem hohen Salzgehalt.

Es wurde nunmehr überraschenderweise gefunden, dass Färbemittel, die neben den farbgebenden Komponenten mindestens einen Fettsäureglycerylester und mindestens ein spezielles kationisches Tensid enthalten, trotz hoher Salzgehalte außerordentlich stabil sind und ferner eine ausgezeichnete Pflegewirkung entfalten.

Ein erster Gegenstand der vorliegenden Anmeldung ist daher ein Mittel zur Färbung keratinischer Fasern, enthaltend
(A) mindestens ein Farbstoffvorprodukt
(B) mindestens einen Fettsäureglycerylester und
(C) mindestens ein kationisches Tensid der Formel (I) 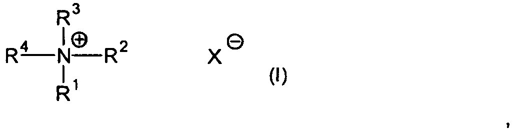
wobei die Reste R¹ bis R⁴ stehen unabhängig voneinander für eine C₁- bis C₄-Alkylkette oder eine C₁₀- bis C₃₀-Alkylkette, mit der Maßgabe, dass mindestens einer und höchstens zwei der Reste R¹ bis R⁴ für eine C₁₂- bis C₃₀-Alkylkette stehen, und X steht für ein physiologisch verträgliches Anion,
enthält, wobei
das Mittel die Fettsäureglycerylester in Mengen von 0,1 bis 10 Gew.% enthält und
das Mittel weiterhin mindestens ein vollständig oder partiell hydriertes Pflanzenöl enthält.

Obwohl prinzipiell die Mitverwendung von klassischen Verdickungsmitteln nicht ausgeschlossen ist, ist es mit der erfindungsgemäßen Cremegrundlage möglich, auf diese Verbindungsklasse vollständig zu verzichten.

Es wurde ferner gefunden, dass die erfindungsgemäße Cremegrundlage ein sehr gutes Aufzugsvermögen für die Farbstoff(vorprodukt)e aufs Haar ermöglicht, einen sehr guten Längenausgleich der Färbung gewährleistet, und die Fasern nach der Behandlung glänzen.

Unter keratinischen Fasern sind dabei erfindungsgemäß Pelze, Wolle, Federn und insbesondere menschliche Haare zu verstehen.

Hinsichtlich der in den erfindungsgemäßen Färbemitteln einsetzbaren weiteren Farbstoffvorprodukte unterliegt die vorliegende Erfindung keinerlei Einschränkungen. Die erfindungsgemäßen Färbemittel können als Farbstoffvorprodukte
- Oxidationsfarbstoffvorprodukte vom Entwickler- und/oder Kuppler-Typ, und
- Vorstufen naturanaloger Farbstoffe, wie Indol- und Indolin-Derivate,
sowie Mischungen von Vertretern dieser Gruppen enthalten.

Im Rahmen einer ersten Ausführungsform der vorliegenden Erfindung enthalten die erfindungsgemäßen Mittel mindestens ein Farbstoffvorprodukt vom Entwickler- und/oder Kupplertyp.

Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Amino-pyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen. Besonders bevorzugt sind p-Phenylendiaminderivate der Formel (E1) 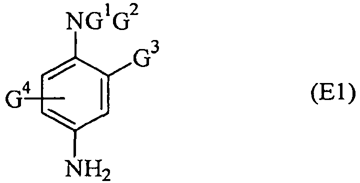 wobei
- G¹ steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest, einen 4'-Aminophenylrest oder einen C₁- bis C₄-Alkylrest, der mit einer stickstoffhaltigen Gruppe, einem Phenyl- oder einem 4'-Aminophenylrest substituiert ist;
- G² steht für ein Wasserstoffatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁-bis C₄)-alkylrest oder einen C₁- bis C₄-Alkylrest, der mit einer stickstoffhaltigen Gruppe substituiert ist;
- G³ steht für ein Wasserstoffatom, ein Halogenatom, wie ein Chlor-, Brom-, lod- oder Fluoratom, einen C₁- bis C₄-Alkylrest, einen C₁- bis C₄-Monohydroxyalkylrest, einen C₂- bis C₄-Polyhydroxyalkylrest, einen C₁- bis C₄-Hydroxyalkoxyrest, einen C₁- bis C₄-Acetylaminoalkoxyrest, einen C₁- bis C₄- Mesylaminoalkoxyrest oder einen C₁- bis C₄-Carbamoylaminoalkoxyrest;
- G⁴ steht für ein Wasserstoffatom, ein Halogenatom oder einen C₁- bis C₄-Alkylrest oder
- wenn G³ und G⁴ in ortho-Stellung zueinander stehen, können sie gemeinsam eine verbrückende α,ω-Alkylendioxogruppe, wie beispielsweise eine Ethylendioxygruppe bilden.

Beispiele für die als Substituenten in den erfindungsgemäßen Verbindungen genannten C₁-bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, Cl-Atome sind ganz besonders bevorzugt. Die weiteren verwendeten Begriffe leiten sich erfindungsgemäß von den hier gegebenen Definitionen ab. Beispiele für stickstoffhaltige Gruppen der Formel (E1) sind insbesondere die Aminogruppen, C₁- bis C₄-Monoalkylaminogruppen, C₁- bis C₄-Dialkylaminogruppen, C₁- bis C₄-Trialkylammoniumgruppen, C₁- bis C₄-Monohydroxyalkylaminogruppen, Imidazolinium und Ammonium.

Besonders bevorzugte p-Phenylendiamine der Formel (E1) sind ausgewählt aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)-anilin, N,N-Bis-(β-hydroxyethyl)-p-phenylendiamin, 4-N,N-Bis-(β-hydroxyethyl)-amino-2-methylanilin, 4-N,N-Bis-(β-hydroxyethyl)-amino-2-chloranilin, 2-(β-Hydroxyethyl)-p-phenylendiamin, 2-(α,β-Dihydroxyethyl)-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl,β-hydroxyethyl)-p-phenylendiamin, N-(β,γ-Dihydroxypropyl)-p-phenylendiamin, N-(4'-Aminophenyl)-p-phenylendiamin, N-Phenyl-p-phenylendiamin, 2-(β-Hydroxyethyloxy)-p-phenylendiamin, 2-(β-Acetylaminoethyloxy)-p-phenylendiamin, N-(β-Methoxyethyl)-p-phenylendiamin und 5,8-Diaminobenzo-1,4-dioxan sowie ihren physiologisch verträglichen Salzen.

Erfindungsgemäß ganz besonders bevorzugte p-Phenylendiaminderivate der Formel (E1) sind p-Phenylendiamin, p-Toluylendiamin, 2-(β-Hydroxyethyl)-p-phenylendiamin, 2-(α,β-Dihydroxyethyl)-p-phenylendiamin und N,N-Bis-(β-hydroxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Unter den zweikernigen Entwicklerkomponenten, die in den Färbezusammensetzungen gemäß der Erfindung verwendet werden können, kann man insbesondere die Verbindungen nennen, die der folgenden Formel (E2) entsprechen, sowie ihre physiologisch verträglichen Salze: 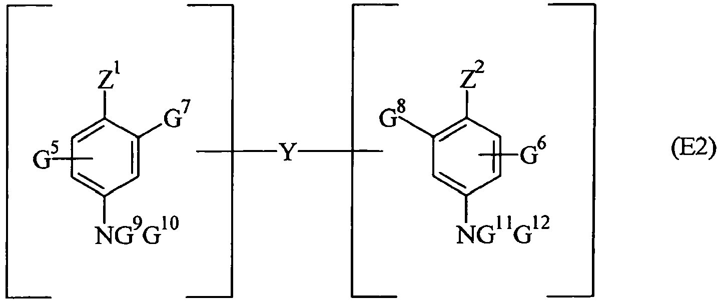 wobei:
- Z¹ und Z² stehen unabhängig voneinander für einen Hydroxyl- oder NH₂-Rest, der gegebenenfalls durch einen C₁- bis C₄-Alkylrest, durch einen C₁- bis C₄-Hydroxyalkylrest und/oder durch eine Verbrückung Y substituiert ist oder der gegebenenfalls Teil eines verbrückenden Ringsystems ist,
- die Verbrückung Y steht für eine Alkylengruppe mit 1 bis 14 Kohlenstoffatomen, wie beispielsweise eine lineare oder verzweigte Alkylenkette oder einen Alkylenring, die von einer oder mehreren stickstoffhaltigen Gruppen und/oder einem oder mehreren Heteroatomen wie Sauerstoff-, Schwefel- oder Stickstoffatomen unterbrochen oder beendet sein kann und eventuell durch einen oder mehrere Hydroxyl- oder C₁- bis C₈-Alkoxyreste substituiert sein kann, oder eine direkte Bindung,
- G⁵ und G⁶ stehen unabhängig voneinander für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen C₁- bis C₄-Aminoalkylrest oder eine direkte Verbindung zur Verbrückung Y,
- G⁷, G⁸, G⁹, G¹⁰, G¹¹ und G¹² stehen unabhängig voneinander für ein Wasserstoffatom, eine direkte Bindung zur Verbrückung Y oder einen C₁- bis C₄-Alkylrest, mit den Maßgaben, dass
- die Verbindungen der Formel (E2) nur eine Verbrückung Y pro Molekül enthalten und
- die Verbindungen der Formel (E2) mindestens eine Aminogruppe enthalten, die mindestens ein Wasserstoffatom trägt.

Die in Formel (E2) verwendeten Substituenten sind erfindungsgemäß analog zu den obigen Ausführungen definiert.

Bevorzugte zweikernige Entwicklerkomponenten der Formel (E2) sind insbesondere: N,N'-Bis-(β-hydroxyethyl)-N,N'-bis-(4'-aminophenyl)-1,3-diamino-propan-2-ol, N,N'-Bis-(β-hydroxyethyl)-N,N'-bis-(4'-aminophenyl)-ethylendiamin, N,N'-Bis-(4-aminophenyl)-tetramethylendiamin, N,N'-Bis-(β-hydroxyethyl)-N,N'-bis-(4-aminophenyl)-tetramethylendiamin, N,N'-Bis-(4-methyl-aminophenyl)-tetramethylendiamin, N,N'-Diethyl-N,N'-bis-(4'-amino-3'-methylphenyl)-ethylendiamin, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan, N,N'-Bis-(2-hydroxy-5-aminobenzyl)-piperazin, N-(4'-Aminophenyl)-p-phenylendiamin und 1,10-Bis-(2',5'-diaminophenyl)-1,4,7,10-tetraoxadecan und ihre physiologisch verträglichen Salze.

Ganz besonders bevorzugte zweikernige Entwicklerkomponenten der Formel (E2) sind N,N'-Bis-(β-hydroxyethyl)-N,N'-bis-(4'-aminophenyl)-1,3-diamino-propan-2-ol, Bis-(2-hydroxy-5-aminophenyl)-methan, 1,3-Bis-(2,5-diaminophenoxy)-propan-2-ol, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Besonders bevorzugt sind p-Aminophenolderivate der Formel (E3) 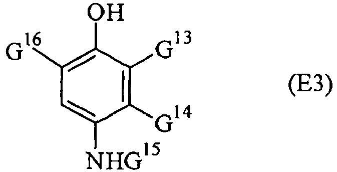 wobei:
- G¹³ steht für ein Wasserstoffatom, ein Halogenatom, einen C₁- bis C₄-Alkylrest, einen C₁- bis C₄-Monohydroxyalkylrest, einen C₂- bis C₄-Polyhydroxyalkylrest, einen (C₁-bis C₄)-Alkoxy-(C₁- bis C₄)-alkylrest, einen C₁- bis C₄-Aminoalkylrest, einen Hydroxy-(C₁-bis C₄)-alkylaminorest, einen C₁- bis C₄-Hydroxyalkoxyrest, einen C₁- bis C₄-Hydroxyalkyl-(C₁-bis C₄)-aminoalkylrest oder einen (Di-C₁- bis C₄-Alkylamino)-(C₁- bis C₄)-alkylrest, und
- G¹⁴ steht für ein Wasserstoff- oder Halogenatom, einen C₁- bis C₄-Alkylrest, einen C₁- bis C₄-Monohydroxyalkylrest, einen C₂- bis C₄-Polyhydroxyalkylrest, einen (C₁- bis C₄)-Alkoxy-(C₁- bis C₄)-alkylrest, einen C₁- bis C₄-Aminoalkylrest oder einen C₁- bis C₄-Cyanoalkylrest,
- G¹⁵ steht für Wasserstoff, einen C₁- bis C₄-Alkylrest, einen C₁- bis C₄-Monohydroxyalkylrest, einen C₂- bis C₄-Polyhydroxyalkylrest, einen Phenylrest oder einen Benzylrest, und
- G¹⁶ steht für Wasserstoff oder ein Halogenatom.

Die in Formel (E3) verwendeten Substituenten sind erfindungsgemäß analog zu den obigen Ausführungen definiert.

Bevorzugte p-Aminophenole der Formel (E3) sind insbesondere p-Aminophenol, N-Methylp-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(β-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(β-hydroxyethyl-aminomethyl)-phenol, 4-Amino-2-(α,β-dihydroxyethyl)-phenol, 4-Amino-2-fluorphenol, 4-Amino-2-chlorphenol, 4-Amino-2,6-dichlorphenol, 4-Amino-2-(diethyl-aminomethyl)-phenol sowie ihre physiologisch verträglichen Salze.

Ganz besonders bevorzugte Verbindungen der Formel (E3) sind p-Aminophenol, 4-Amino-3-methylphenol, 4-Amino-2-aminomethylphenol, 4-Amino-2-(α,β-dihydroxyethyl)-phenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen.

Bevorzugte Pyridin-Derivate sind insbesondere die Verbindungen, die in den Patenten GB 1 026 978 und GB 1 153 196 beschrieben werden, wie 2,5-Diamino-pyridin, 2-(4'-Methoxyphenyl)-amino-3-amino-pyridin, 2,3-Diamino-6-methoxy-pyridin, 2-(β-Methoxyethyl)-amino-3-amino-6-methoxy-pyridin und 3,4-Diamino-pyridin.

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin.

Bevorzugte Pyrazol-Derivate sind insbesondere die Verbindungen, die in den Patenten DE 3 843 892, DE 4 133 957 und Patentanmeldungen WO 94/08969, WO 94/08970, EP-740 931 und DE 195 43 988 beschrieben werden, wie 4,5-Diamino-1-methylpyrazol, 4,5-Diamino-1-(β-hydroxyethyl)-pyrazol, 3,4-Diaminopyrazol, 4,5-Diamino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxyethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(β-aminoethyl)-amino-1,3-dimethylpyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Bevorzugte Pyrazol-Pyrimidin-Derivate sind insbesondere die Derivate des Pyrazol-[1,5-a]-pyrimidin der folgenden Formel (E4) und dessen tautomeren Formen, sofern ein tautomeres Gleichgewicht besteht: 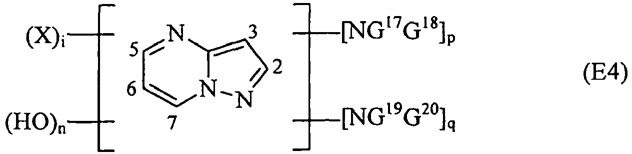 wobei:
- G¹⁷, G¹⁸, G¹⁹ und G²⁰ unabhängig voneinander stehen für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest einen (C₁- bis C₄)-Alkoxy-(C₁- bis C₄)-alkylrest, einen C₁- bis C₄-Aminoalkylrest, der gegebenenfalls durch ein Acetyl-Ureid- oder einen Sulfonyl-Rest geschützt sein kann, einen (C₁- bis C₄)-Alkylamino-(C₁- bis C₄)-alkylrest, einen Di-[(C₁- bis C₄)-alkyl]-(C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄)-[Hydroxyalkyl]-(C₁- bis C₄)-aminoalkylrest,
- die X-Reste stehen unabhängig voneinander für ein Wasserstoffatom, einen C₁- bis C₄-Alkylrest, einen Aryl-Rest, einen C₁- bis C₄-Hydroxyalkylrest, einen C₂- bis C₄-Polyhydroxyalkylrest, einen C₁- bis C₄-Aminoalkylrest, einen (C₁- bis C₄)-Alkylamino-(C₁-bis C₄)-alkylrest, einen Di-[(C₁- bis C₄)alkyl]- (C₁- bis C₄)-aminoalkylrest, wobei die Dialkyl-Reste gegebenenfalls einen Kohlenstoffzyklus oder einen Heterozyklus mit 5 oder 6 Kettengliedern bilden, einen C₁- bis C₄-Hydroxyalkyl- oder einen Di-(C₁- bis C₄-hydroxyalkyl)-aminoalkylrest, einen Aminorest, einen C₁- bis C₄-Alkyl- oder Di-(C₁- bis C₄-hydroxyalkyl)-aminorest, ein Halogenatom, eine Carboxylsäuregruppe oder eine Sulfonsäuregruppe,
- i hat den Wert 0, 1, 2 oder 3,
- p hat den Wert 0 oder 1,
- q hat den Wert 0 oder 1 und
- n hat den Wert 0 oder 1,
mit der Maßgabe, dass
- die Summe aus p + q ungleich 0 ist,
- wenn p + q gleich 2 ist, n den Wert 0 hat, und die Gruppen NG¹⁷G¹⁸ und NG¹⁹G²⁰ belegen die Positionen (2,3); (5,6); (6,7); (3,5) oder (3,7);
- wenn p + q gleich 1 ist, n den Wert 1 hat, und die Gruppen NG¹⁷G¹⁸ (oder NG¹⁹G²⁰) und die Gruppe OH belegen die Positionen (2,3); (5,6); (6,7); (3,5) oder (3,7);

Die in Formel (E4) verwendeten Substituenten sind erfindungsgemäß analog zu den obigen Ausführungen definiert.

Wenn das Pyrazol-[1,5-a]-pyrimidin der obenstehenden Formel (E4) eine Hydroxygruppe an einer der Positionen 2, 5 oder 7 des Ringsystems enthält, besteht ein tautomeres Gleichgewicht, das zum Beispiel im folgenden Schema dargestellt wird:

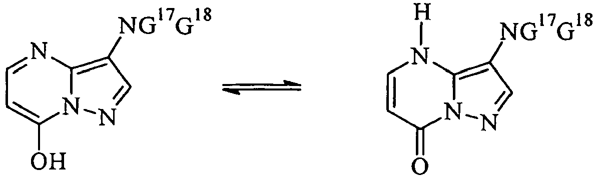

Unter den Pyrazol-[1,5-a]-pyrimidinen der obenstehenden Formel (E4) kann man insbesondere nennen:
- Pyrazol-[1,5-a]-pyrimidin-3,7-diamin;
- 2,5-Dimethyl-pyrazol-[1,5-a]-pyrimidin-3,7-diamin;
- Pyrazol-[1,5-a]-pyrimidin-3,5-diamin;
- 2,7-Dimethyl-pyrazol-[1,5-a]-pyrimidin-3,5-diamin;
- 3-Aminopyrazol-[1,5-a]-pyrimidin-7-ol;
- 3-Aminopyrazol-[1,5-a]-pyrimidin-5-ol;
- 2-(3-Aminopyrazol-[1,5-a]-pyrimidin-7-ylamino)-ethanol;
- 2-(7-Aminopyrazol-[1,5-a]-pyrimidin-3-ylamino)-ethanol;
- 2-[(3-Aminopyrazol-[1,5-a]-pyrimidin-7-yl)-(2-hydroxy-ethyl)-amino]-ethanol;
- 2-[(7-Aminopyrazol-[1,5-a]-pyrimidin-3-yl)-(2-hydroxy-ethyl)-amino]-ethanol;
- 5,6-Dimethylpyrazol-[1,5-a]-pyrimidin-3,7-diamin;
- 2,6-Dimethylpyrazol-[1,5-a]-pyrimidin-3,7-diamin;
- 3-Amino-7-dimethylamino-2,5-dimethylpyrazol-[1,5-a]-pyrimidin;
sowie ihre physiologisch verträglichen Salze und ihre tautomeren Formen, wenn ein tautomers Gleichgewicht vorhanden ist.

Die Pyrazol-[1,5-a]-pyrimidine der obenstehenden Formel (E4) können wie in der Literatur beschrieben durch Zyklisierung ausgehend von einem Aminopyrazol oder von Hydrazin hergestellt werden.

In einer weiteren bevorzugten Ausführungsform enthalten die erfindungsgemäßen Färbemittel mindestens eine Kupplerkomponente.

Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methyl-pyrazolon-5, 2,4-Dichlor-3-aminophenol, 1,3-Bis-(2',4'-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Erfindungsgemäß bevorzugte Kupplerkomponenten sind
- m-Aminophenol und dessen Derivate wie beispielsweise 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)-amino-2-methylphenol, 3-(Diethylamino)-phenol, N-Cyclopentyl-3-aminophenol, 1,3-Dihydroxy-5-(methylamino)-benzol, 3-Ethylamino-4-methylphenol und 2,4-Dichlor-3-aminophenol,
- o-Aminophenol und dessen Derivate,
- m-Diaminobenzol und dessen Derivate wie beispielsweise 2,4-Diaminophenoxyethanol, 1,3-Bis-(2',4'-diaminophenoxy)-propan, 1-Methoxy-2-amino-4-(2'-hydroxyethylamino)benzol, 1,3-Bis-(2',4'-diaminophenyl)-propan, 2,6-Bis-(2'-hydroxyethylamino)-1-methylbenzol und 1-Amino-3-bis-(2'-hydroxyethyl)-aminobenzol,
- o-Diaminobenzol und dessen Derivate wie beispielsweise 3,4-Diaminobenzoesäure und 2,3-Diamino-1-methylbenzol,
- Di- beziehungsweise Trihydroxybenzolderivate wie beispielsweise Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol,
- Pyridinderivate wie beispielsweise 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin und 3,5-Diamino-2,6-dimethoxypyridin,
- Naphthalinderivate wie beispielsweise 1-Naphthol, 2-Methyl-1-naphthol, 2-Hydroxymethyl-1-naphthol, 2-Hydroxyethyl-1-naphthol, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin,
- Morpholinderivate wie beispielsweise 6-Hydroxybenzomorpholin und 6-Amino-benzomorpholin,
- Chinoxalinderivate wie beispielsweise 6-Methyl-1,2,3,4-tetrahydrochinoxalin,
- Pyrazolderivate wie beispielsweise 1-Phenyl-3-methylpyrazol-5-on,
- Indolderivate wie beispielsweise 4-Hydroxyindol, 6-Hydroxyindol und 7-Hydroxyindol,
- Pyrimidinderivate, wie beispielsweise 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin, oder
- Methylendioxybenzolderivate wie beispielsweise 1-Hydroxy-3,4-methylendioxybenzol, 1-Amino-3,4-methylendioxybenzol und 1-(2'-Hydroxyethyl)-amino-3,4-methylendioxybenzol.

Erfindungsgemäß besonders bevorzugte Kupplerkomponenten sind 1-Naphthol, 1,5-, 2,7-und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Die erfindungsgemäßen Färbemittel enthalten sowohl die Entwicklerkomponenten als auch die Kupplerkomponenten bevorzugt in einer Menge von 0,005 bis 20 Gew.-%, vorzugsweise 0,1 bis 5 Gew.-%, jeweils bezogen auf das gesamte Oxidationsfärbemittel. Dabei werden Entwicklerkomponenten und Kupplerkomponenten im allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1:0,5 bis 1:3, insbesondere 1:1 bis 1:2, enthalten sein können.

In einer weiteren Ausführungsform der vorliegenden Erfindung enthalten die Färbemittel als Farbstoffvorprodukt (FV) mindestens eine Vorstufe eines naturanalogen Farbstoffs. Als Vorstufen naturanaloger Farbstoffe werden bevorzugt solche Indole und Indoline eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders gut als Vorstufen naturanaloger Haarfarbstoffe geeignet sind Derivate des 5,6-Dihydroxyindolins der Formel (IIa), 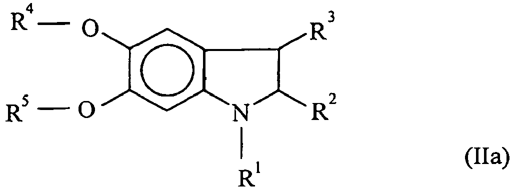 in der unabhängig voneinander
- R¹ steht für Wasserstoff, eine C₁-C₄-Alkylgruppe oder eine C₁-C₄-Hydroxy-alkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
sowie physiologisch verträgliche Salze dieser Verbindungen mit einer organischen oder anorganischen Säure.

Besonders bevorzugte Derivate des Indolins sind das 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin, 5,6-Dihydroxyindolin-2-carbon-säure sowie das 6-Hydroxyindolin, das 6-Aminoindolin und das 4-Aminoindolin.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Als Vorstufen naturanaloger Haarfarbstoffe hervorragend geeignet sind weiterhin Derivate des 5,6-Dihydroxyindols der Formel (IIb), 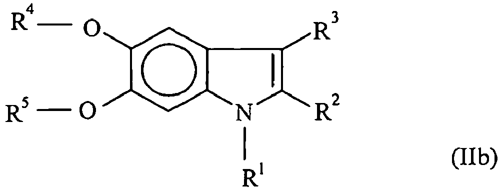 in der unabhängig voneinander
- R¹ steht für Wasserstoff, eine C₁-C₄-Alkylgruppe oder eine C₁-C₄-Hydroxyalkylgruppe,
- R² steht für Wasserstoff oder eine -COOH-Gruppe, wobei die -COOH-Gruppe auch als Salz mit einem physiologisch verträglichen Kation vorliegen kann,
- R³ steht für Wasserstoff oder eine C₁-C₄-Alkylgruppe,
- R⁴ steht für Wasserstoff, eine C₁-C₄-Alkylgruppe oder eine Gruppe -CO-R⁶, in der R⁶ steht für eine C₁-C₄-Alkylgruppe, und
- R⁵ steht für eine der unter R⁴ genannten Gruppen,
- sowie physiologisch verträgliche Salze dieser Verbindungen mit einer organischen oder anorganischen Säure.

Besonders bevorzugte Derivate des Indols sind 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, 6-Hydroxyindol, 6-Aminoindol und 4-Aminoindol.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den erfindungsgemäßen Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Färbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin und Histidin, insbesondere Arginin.

Die erfindungsgemäße Cremegrundlage hat sich ferner als besonders geeignet für Färbungen auf Basis von direktziehenden Farbstoffen erwiesen. Neben den erfindungsgemäßen Farbstoffvorprodukten können die erfindungsgemäßen Färbemittel daher in einer weiteren bevorzugten Ausführungsform der vorliegenden Erfindung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

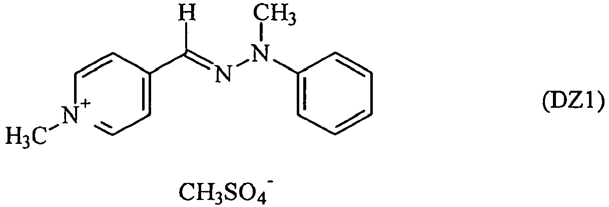

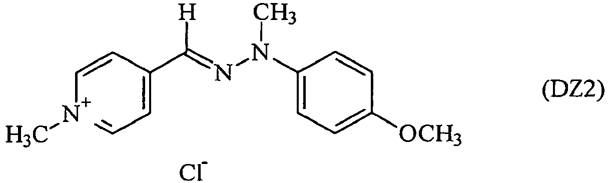

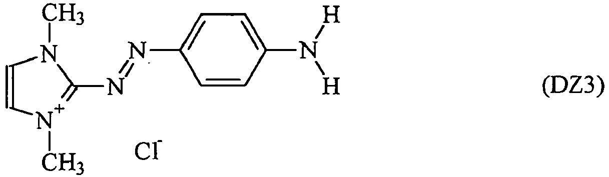

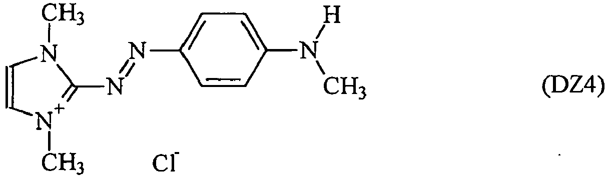

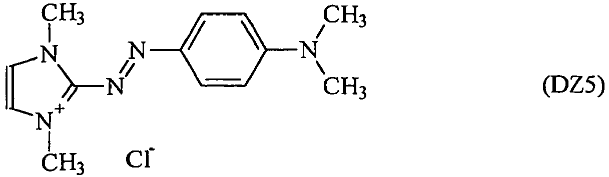

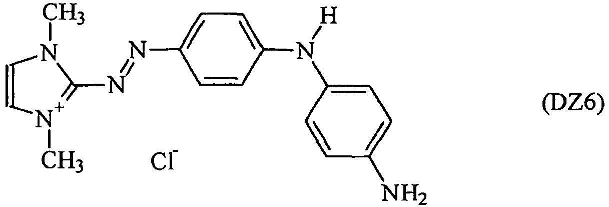

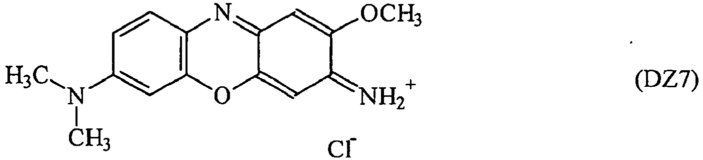

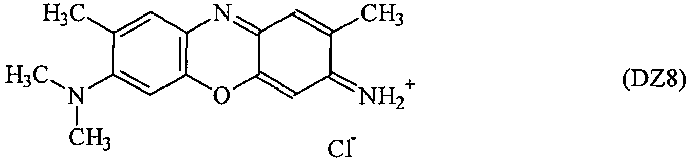

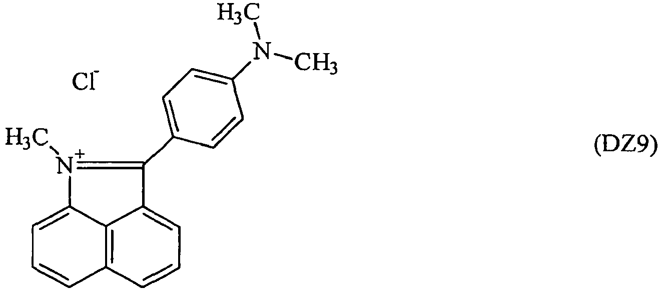

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York, Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Als zweite erfindungswesentliche Komponente enthalten die Färbemittel mindestens 0.1 bis 10 Gew.-% Fettsäureglycerylester. Erfindungsgemäß bevorzugte Fettsäureglycerylester sind die sogenannten Fettsäurepartialglyceride, insbesondere Monoglyceride, Diglyceride und deren technische Gemische. Bei der Verwendung technischer Produkte können herstellungsbedingt noch geringe Mengen Triglyceride enthalten sein.

Bevorzugte Fettsäureglycerylester sind daher die Verbindungen der Formel (III), 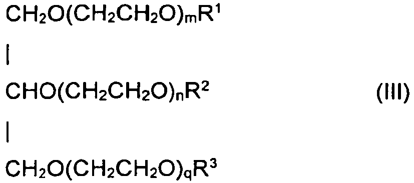 in der R¹, R² und R³ unabhängig voneinander für Wasserstoff oder für einen linearen oder verzweigten, gesättigten und/oder ungesättigten Acylrest mit 6 bis 22, vorzugsweise 12 bis 18, Kohlenstoffatomen stehen mit der Maßgabe, dass mindestens eine dieser Gruppen für einen Acylrest und mindestens eine dieser Gruppen für Wasserstoff steht. Die Summe (m+n+q) steht für 0 oder Zahlen von 1 bis 100, vorzugsweise für 0 oder 5 bis 25. Ganz besonders bevorzugt sind die Verbindungen der Formel (III), bei denen die Summe (m+n+q) für 0 steht. Besonders bevorzugt sind Verbindungen der Formel (III), bei denen R¹ für einen Acylrest und R² und R³ für Wasserstoff stehen. Typische Beispiele sind Mono- und/oder Diglyceride auf Basis von Adipinsäure, Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen. Vorzugsweise werden Laurinsäuremonoglyceride, Isostearinsäuremonoglyceride, Behensäuremonoglycerid, Linolsaäuremonoglyceride, Ölsäuremonoglyceride und Stearinsäuremonoglyceride eingesetzt. Ölsäuremonoglyceride und Stearinsäuremonoglyceride sind erfindungsgemäß ganz besonders bevorzugte Fettsäuregylcerylester.

Die Fettsäureglycerylester sind in den erfindungsgemäßen Mitteln in Mengen von 0,1 bis 10 Gew.-%, insbesondere von 0,3 bis 3 Gew.-% enthalten.

Als dritte obligatorische Komponente enthalten die erfindungsgemäßen Mittel mindestens ein spezielles kationisches Tensid der Formel (I) 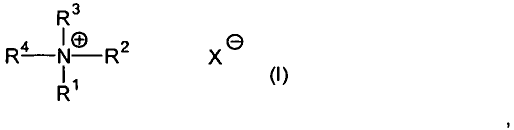 wobei die Reste R¹ bis R⁴ stehen unabhängig voneinander für eine C₁- bis C₄-Alkylkette oder eine C₁₀- bis C₃₀-Alkylkette, mit der Maßgabe, dass mindestens einer und höchstens zwei der Reste R¹ bis R⁴ für eine C₁₂- bis C₃₀-Alkylkette stehen, und X steht für ein physiologisch verträgliches Anion.

Bevorzugte C₁- bis C₄-Alkylketten im Sinne der Formel (I) sind Methyl-, Ethyl-, Propyl-, Isopropyl- und Butylgruppen, die Methylgruppe ist erfindungsgemäß besonders bevorzugt.

Bevorzugte C₁₀- bis C₃₀-Alkylketten im Sinne der Formel (I) sind die Caprin-, Cetyl-, Stearyl-, Behenyl- und Laurylgruppen. Erfindungsgemäß können C₁₂- bis C₃₀-Alkylketten besonders bevorzugt sein. Die Cetyl- und die Behenylgruppen sind erfindungsgemäß ganz besonders bevorzugt.

Bevorzugte physiologisch verträgliche Anionen X sind im Sinne der vorliegenden Erfindung sowohl anorganische Anionen, wie beispielsweise die Halogenide (Chlorid, Bromid, Iodid), das Sulfat und das Phosphat, als auch organische Anionen, wie beispielsweise das Acetat und das Lactat. Ein weiteres Anion im Sinne der vorliegenden Erfindung ist das Methosulfatanion. Das Chlorid und das Bromid sind erfindungsgemäß besonders bevorzugt, ganz besonders bevorzugt ist erfindungsgemäß das Chlorid.

Bevorzugte Verbindungen der Formel (I) sind die Alkyltrimethylammoniumchloride, Dialkyldimethylammoniumchloride, Alkyltrimethylammoniumbromide und Dialkyldimethylammoniumbromide. Ganz besonders bevorzugte Verbindungen der Formel (I) sind Dicaprindimethylammoniumchlorid, Lauryltrimethylammoniumchlorid, Cetyltrimethylammoniumchlorid, Stearyltrimethylammoniumchlorid, Distearyldimethylammoniumchlorid, Dicetyldimethylammoniumchlorid, Dilauryldimethylammoniumchlorid, Behenyltrimethylammoniumchlorid sowie Behenyltrimethylammoniummethosulfat. Weiterhin können auch Mischung der genannten Verbindungen, insbesondere natürlich vorkommende Mischungen, wie beispielweise die Derivate des Talgfettalkohols, erfindungsgemöß bevorzugt sein. Als Vertreter seien an dieser Stelle Ditallowdimethylammoniumchlorid und Monotallowtrimethylammoniumchlorid explizit genannt.

Die kationischen Tenside der Formel (I) sind in den erfindungsgemäß verwendeten Mitteln bevorzugt in Mengen von 0,05 bis 10 Gew.-%, bezogen auf das gesamte Mittel, enthalten. Mengen von 0,1 bis 5 Gew.-% sind besonders bevorzugt.

Weiterhin enthalten die Färbemittel mindestens ein vollständig oder partiell hydriertes Pflanzenöl. Erfindungsgemäß bevorzugte Pflanzenöle sind beispielsweise Sojabohnenöl, Kokosnussöl, Haselnussöl, Palmkernöl, Sonnenblumenöl, Leinsamenöl, Erdnussöl, Rizinusöl oder Rapsöl.

Ein erfindungsgemäße ganz besonders bevorzugtes hydriertes Pflanzenöl ist das unter der Handelsbezeichnung Cutina^{®} HR (Cognis) vertriebene hydrierte Rizinusöl.

Die erfindungsgemäßen Mittel enthalten die vollständig oder partiell hydrierten Pflanzenöle bevorzugt in Mengen von 0,1 bis 7 Gew.-%, ein Mengenbereich von 0,4 bis 1,2 Gew.-% ist besonders bevorzugt.

In einer weiteren bevorzugten Ausführungsform der vorliegenden Erfindung enthalten die Färbemittel Ammoniumhydrogencarbonat.

Ammoniumhydrogencarbonat, (NH₄)HCO₃, (auch als Ammoniumbicarbonat oder doppeltkohlensaures Ammonium bezeichnet) kommt in Form großer glänzender, harter, farbloser, fast geruchloser Prismen in den Handel. Bevorzugte erfindungsgemäße Mittel enthalten- bezogen auf das Gewicht des Mittels - 0,1 bis 10 Gew.-%, vorzugsweise 0,25 bis 8 Gew.-%, besonders bevorzugt 0,5 bis 5 Gew.-% und insbesondere 1 bis 3 Gew.-% Ammoniumhydrogencarbonat.

In einer weiteren bevorzugten Ausführungsform der vorliegenden Erfindung enthalten die Färbemittel mindestens eine Kieselsäure.

"Kieselsäure" ist im Rahmen der vorliegenden Anmeldung die Sammelbezeichnung für Verbindungen der allgemeinen Formel (SiO₂)ₘ · nH₂O. Setzt man ein Silicium halogenid (z.B. SiCl₄) mit Wasser um, so bildet sich primär die *Orthokieselsäure* (Monokieselsäure), H₄SiO₄, die nur in großer Verdünnung beständig und intermolekular Wasser abspaltet. Als erstes Kondensationsprodukt tritt so die *Dikieselsäure* (Pyrokieselsäure, H₆Si₂O₇) auf. Weitere Kondensation führt auf dem Weg über cyclische Kieselsäuren und käfigartige

Kieselsäuren zu annähernd kugelförmigen *Polykieselsäuren.* Formales Endprodukt der Kondensation ist polymeres Siliciumdioxid, (SiO₂)ₓ, das Anhydrid der Kieselsäure. Bei der Kondensation laufen kettenverlängernde, ringbildende und verzweigende Prozesse nebeneinander ab, so daß die Polykieselsäuren ungeordnet aufgebaut (amorph) sind. Die Si-Atome befinden sich bei allen Kieselsäuren im Mittelpunkt von unregelmäßig miteinander verknüpften Tetraedern, an deren 4 Eckpunkten O-Atome liegen, die gleichzeitig den Nachbartetraedern angehören.

Kieselsäuren können aus der Lösung ("Fällungskieselsäuren") oder durch Pyrolyse von flüchtigen Si-Verbindungen hergestellt werden ("pyrogene Kieselsäuren"). Hinsichtlich des Produktionsumfanges haben die Fällungskieselsäuren die bei weitem größte Bedeutung. Sie werden aus einer wäßrigen Alkalisilicat-Lösung durch Fällung mit Mineralsäuren hergestellt. Dabei bilden sich kolloidale Primärteilchen, die mit fortschreitender Reaktion agglomerieren und schließlich zu Aggregaten verwachsen. Die pulverförmigen, voluminösen Formen besitzen Porenvolumina von 2,5 -15 mL/g und spezifische Oberflächen von 30-800 m²/g.

Unter der Bezeichnung pyrogene Kieselsäuren. werden hochdisperse Kieselsäuren zusammengefaßt, die durch Flammenhydrolyse hergestellt werden. Dabei wird Siliciumtetrachlorid in einer Knallgas-Flamme zersetzt. Pyrogene Kieselsäuren besitzen an ihrer nahezu porenfreien Oberfläche deutlich weniger OH-Gruppen als Fällungskieselsäuren Wegen ihrer durch die Silanol-Gruppen bedingten Hydrophilie werden die synthet. Kieselsäuren häufig chemischen Nachbehandlungsverfahren unterzogen, bei denen die OH-Gruppen z.B. mit organischen Chlorsilanen reagieren. Dadurch entstehen modifizierte, z.B. hydrophobe Oberflächen, welche die anwendungstechnischen Eigenschaften der Kieselsäuren wesentlich erweitern. Im Rahmen der vorliegenden Erfindung sind sowohl Fällungs- als auch pyrogene Kieselsäuren einsetzbar. Bevorzugt einsetzbare Kieselsäuren weisen spezifische Oberflächen von 25 bis 1000 m²/g, Ölzahlen von 30 bis 300 g/100 g, Primärpartikelgrößen von 5 bis 500 nm, Dichten von 1,9 bis 2,2 g/cm³ und Trocknungsverlust < 7 %, vorzugsweise < 3 % auf. Im Rahmen der vorliegenden Erfindung einsetzbare handelsprodukte werden beispielsweise unter den warenzeichen Aerosil^{®}, Cab-O-Sil^{®} und HDK vertreiben.

Erfindungsgemäß bevorzugte Mittel sind dadurch gekennzeichnet, daß sie - bezogen auf das Gewicht des Mittels - 0,05 bis 5 Gew.-%, vorzugsweise 0,1 bis 3 Gew.-%, besonders bevorzugt 0,15 bis 2 Gew.-% und insbesondere 0,2 bis 1 Gew.-% Kieselsäure, enthalten. Der Einsatz von Ammoniumhydrogencarbonat und/oder Kieselsäure führt dabei zu einer konstant guten Farbleistung der Färbecreme sowohl über längere Lagerungs- als auch über längere Anwendungszeiträume hinweg.

In einer weiteren bevorzugten Ausführungsform der vorliegenden Erfindung enthalten die Färbemittel weiterhin mindestens ein nichtionogenes Tensid. Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und -oligoglycoside und deren ethoxylierte Analoga sowie
- Anlagerungsprodukte von 5 bis 60 Mol Ethylenoxid an Rizinusöl und gehärtetes Rizinusöl.

Bevorzugte nichtionische Tenside sind Alkylpolyglykoside der allgemeinen Formel R¹O-(Z)_{X}. Diese Verbindungen sind durch die folgenden Parameter gekennzeichnet.

Der Alkylrest R¹ enthält 6 bis 22 Kohlenstoffatome und kann sowohl linear als auch verzweigt sein. Bevorzugt sind primäre lineare und in 2-Stellung methylverzweigte aliphatische Reste. Solche Alkylreste sind beispielsweise 1-Octyl, 1-Decyl, 1-Lauryl, 1-Myristyl, 1-Cetyl und 1-Stearyl. Besonders bevorzugt sind 1-Octyl, 1-Decyl, 1-Lauryl, 1-Myristyl. Bei Verwendung sogenannter "Oxo-Alkohole" als Ausgangsstoffe überwiegen Verbindungen mit einer ungeraden Anzahl von Kohlenstoffatomen in der Alkylkette.

Die erfindungsgemäß verwendbaren Alkylpolyglykoside können beispielsweise nur einen bestimmten Alkylrest R¹ enthalten. Üblicherweise werden diese Verbindungen aber ausgehend von natürlichen Fetten und Ölen oder Mineralölen hergestellt. In diesem Fall liegen als Alkylreste R Mischungen entsprechend den Ausgangsverbindungen bzw. entsprechend der jeweiligen Aufarbeitung dieser Verbindungen vor.

Besonders bevorzugt sind solche Alkylpolyglykoside, bei denen R¹
- im wesentlichen aus C₈- und C₁₀-Alkylgruppen,
- im wesentlichen aus C₁₂- und C₁₄-Alkylgruppen,
- im wesentlichen aus C₈- bis C₁₆-Alkylgruppen oder
- im wesentlichen aus C₁₂- bis C₁₆-Alkylgruppen besteht.

Als Zuckerbaustein Z können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Die erfindungsgemäß verwendbaren Alkylpolyglykoside enthalten im Schnitt 1,1 bis 5 Zuckereinheiten. Alkylpolyglykoside mit x-Werten von 1,1 bis 1,6 sind bevorzugt. Ganz besonders bevorzugt sind Alkylglykoside, bei denen x 1,1 bis 1,4 beträgt.

Die Alkylglykoside können neben ihrer Tensidwirkung auch dazu dienen, die Fixierung von Duftkomponenten auf dem Haar zu verbessern. Der Fachmann wird also für den Fall, dass eine über die Dauer der Haarbehandlung hinausgehende Wirkung des Parfümöles auf dem Haar gewünscht wird, bevorzugt zu dieser Substanzklasse als weiterem Inhaltsstoff der erfindungsgemäßen Zubereitungen zurückgreifen.

Auch die alkoxylierten Homologen der genannten Alkylpolyglykoside können erfindungsgemäß eingesetzt werden. Diese Homologen können durchschnittlich bis zu 10 Ethylenoxid- und/oder Propylenoxideinheiten pro Alkylglykosideinheit enthalten.

Erfindungsgemäß bevorzugte nichtionogene Tenside sind die Anlagerungsprodukte von 2 bis 30 Mol Ethylenoxid und/oder 0 bis 5 Mol Propylenoxid an lineare Fettalkohole mit 8 bis 22 C-Atomen. Bei diesen Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Es hat sich erfindungsgemäß als besonders bevorzugt erwiesen, zwei verschiedene Anlagerungsprodukte von Ethylenoxid an lineare Fettalkohole einzusetzen. Auf diese Weise kann die Stabilität sowie die Homogenität der Emulsionen noch weiter verbessert werden.

Im Rahmen einer ersten bevorzugten Ausführungsform wird daher mindestens ein Anlagerungsprodukte von Ethylenoxid an einen lineare Fettalkohol mit einer Kettenlänge von höchstens 14 Kohlenstoffatomen und mindestens ein Anlagerungsprodukte von Ethylenoxid an lineare Fettalkohole mit einer Kettenlänge von mindestens 15 Kohlenstoffatomen eingesetzt. Eine Kombination aus einem Anlagerungsprodukt von Ethylenoxid an einen lineare Fettalkohol mit einer Kettenlänge von 10-14 Kohlenstoffatomen mit einem Anlagerungsprodukt von Ethylenoxid an lineare Fettalkohole mit einer Kettenlänge von 15-22 Kohlenstoffatomen kann erfindungsgemäß besonders bevorzugt sein.

In einer weiteren bevorzugten Ausführungsform wird mindestens ein Anlagerungsprodukt von Ethylenoxid an lineare Fettalkohole mit einem durchsschnittlichen Ethoxylierungsgrad von höchstens 25 EO-Einheiten und mindestens ein Anlagerungsprodukt von Ethylenoxid an lineare Fettalkohole mit einem durchsschnittlichen Ethoxylierungsgrad von mindestens 28 EO-Einheiten eingesetzt. Eine Kombination von einem Anlagerungsprodukt von Ethylenoxid an lineare Fettalkohole mit einem durchsschnittlichen Ethoxylierungsgrad von 18-25 EO-Einheiten mit einem Anlagerungsprodukt von Ethylenoxid an lineare Fettalkohole mit einem durchsschnittlichen Ethoxylierungsgrad von mindestens 28-35 EO-Einheiten kann erfindungsgemäß ebenfalls besonders bevorzugte sein.

Eine Kombination von Laureth-23 und Ceteareth-30 hat sich als erfindungsgemäß ganz besonders bevorzugt erwiesen.

Die nichtionogenen Tenside werden bevorzugt in einer Menge von 0,05 bis 5,0 Gew.-%, insbesondere von 0,1 bis 2,0 Gew.-%, bezogen auf das anwendungsbereite Mittel eingesetzt.

In einer bevorzugten Ausführungsform der Erfindung kann die Wirkung des Färbemittels durch Fettstoffe (D) weiter gesteigert werden. Unter Fettstoffen sind erfindungsgemäß Fettsäuren, Fettalkohole, natürliche und synthetische Wachse, welche sowohl in fester Form als auch flüssig in wässriger Dispersion vorliegen können, sowie natürliche und synthetische kosmetische Ölkomponenten zu verstehen.

Als Fettsäuren (D1) können lineare und/oder verzweigte, gesättigte und/oder ungesättigte Fettsäuren mit 6 - 30 Kohlenstoffatomen eingesetzt werden. Bevorzugt sind Fettsäuren mit 10 - 22 Kohlenstoffatomen. Hierunter wären beispielsweise zu nennen die Isostearinsäuren, wie die Handelsprodukte Emersol^{®} 871 und Emersol^{®} 875, und Isopalmitinsäuren wie das Handelsprodukt Edenor^{®} IP 95, sowie alle weiteren unter den Handelsbezeichnungen Edenor^{®} (Cognis) vertriebenen Fettsäuren. Weitere typische Beispiele für solche Fettsäuren sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Besonders bevorzugt sind üblicherweise die Fettsäureschnitte, welche aus Cocosöl oder Palmöl erhältlich sind; insbesondere bevorzugt ist in der Regel der Einsatz von Stearinsäure.

Die Einsatzmenge beträgt dabei 0,1 - 15 Gew.%, bezogen auf das gesamte Mittel. Bevorzugt beträgt die Menge 0,5 - 10 Gew.%, wobei ganz besonders vorteilhaft Mengen von 1 - 5 Gew.% sein können.

Als Fettalkohole (D2) können gesättigte, ein- oder mehrfach ungesättigte, verzweigte oder unverzweigte Fettalkohole mit C₆ - C₃₀-, bevorzugt C₁₀ - C₃₀- und ganz besonders bevorzugt C₁₂ - C₂₂- Kohlenstoffatomen eingesetzt werden. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbetalkohole, wobei diese Aufzählung beispielhaften und nicht limitierenden Charakter haben soll. Die Fettalkohole stammen jedoch von bevorzugt natürlichen Fettsäuren ab, wobei üblicherweise von einer Gewinnung aus den Estern der Fettsäuren durch Reduktion ausgegangen werden kann. Erfindungsgemäß einsetzbar sind ebenfalls solche Fettalkoholschnitte, die durch Reduktion natürlich vorkommender Triglyceride wie Rindertalg, Palmöl, Erdnußöl, Rüböl, Baumwollsaatöl, Sojaöl, Sonnenblumenöl und Leinöl oder aus deren Umesterungsprodukten mit entsprechenden Alkoholen entstehenden Fettsäureestern erzeugt werden, und somit ein Gemisch von unterschiedlichen Fettalkoholen darstellen. Solche Substanzen sind beispielsweise unter den Bezeichnungen Stenol^{®}, z.B. Stenol^{®} 1618 oder Lanette^{®}, z.B. Lanette^{®} O oder Lorol^{®}, z.B. Lorol^{®} C8, Lorol^{®} C14, Lorol^{®} C18, Lorol^{®} C8-18, HD-Ocenol^{®}, Crodacol^{®}, z.B. Crodacol^{®} CS, Novol^{®}, Eutanol^{®} G, Guerbitol^{®} 16, Guerbitol^{®} 18, Guerbitol^{®} 20, Isofol^{®} 12, Isofol^{®} 16, Isofol^{®} 24, Isofol^{®} 36, Isocarb^{®} 12, Isocarb^{®} 16 oder Isocarb^{®} 24 käuflich zu erwerben. Selbstverständlich können erfindungsgemäß auch Wollwachsalkohole, wie sie beispielsweise unter den Bezeichnungen Corona^{®}, White Swan^{®}, Coronet^{®} oder Fluilan^{®} käuflich zu erwerben sind, eingesetzt werden.

Die Fettalkohole (D2) werden in Mengen von 0,1 - 30 Gew.-%, bezogen auf die gesamte Zubereitung, bevorzugt in Mengen von 0,1 - 20 Gew.-% eingesetzt.

Als natürliche oder synthetische Wachse (D3) können erfindungsgemäß eingesetzt werden feste Paraffine oder Isoparaffine, Carnaubawachse, Bienenwachse, Candelillawachse, Ozokerite, Ceresin, Walrat, Sonnenblumenwachs, Fruchtwachse wie beispielsweise Apfelwachs oder Citruswachs, Microwachse aus PE- oder PP. Derartige Wachse sind beispielsweise erhältlich über die Fa. Kahl & Co., Trittau.

Die Einsatzmenge der Wachse (D3) beträgt 0,1 - 50 Gew.% bezogen auf das gesamte Mittel, bevorzugt 0,1 - 20 Gew.% und besonders bevorzugt 0,1 - 15 Gew.% bezogen auf das gesamte Mittel.

Zu den natürlichen und synthetischen kosmetischen Ölkörpern (D4), welche die Wirkung des erfindungsgemäßen Färbemittel steigern können, sind beispielsweise zu zählen:
- pflanzliche Öle. Beispiele für solche Öle sind Sonnenblumenöl, Olivenöl, Sojaöl, Rapsöl, Mandelöl, Jojobaöl, Orangenöl, Weizenkeimöl, Pfirsichkernöl und die flüssigen Anteile des Kokosöls. Geeignet sind aber auch andere Triglyceridöle wie die flüssigen Anteile des Rindertalgs sowie synthetische Triglyceridöle.
- flüssige Paraffinöle, Isoparaffinöle und synthetische Kohlenwasserstoffe sowie Din-alkylether mit insgesamt zwischen 12 bis 36 C-Atomen, insbesondere 12 bis 24 C-Atomen, wie beispielsweise Di-n-octylether, Di-n-decylether, Di-n-nonylether, Din-undecylether, Di-n-dodecylether, n-Hexyl-n-octylether, n-Octyl-n-decylether, n-Decyl-n-undecylether, n-Undecyl-n-dodecylether und n-Hexyl-n-Undecylether sowie Di-tert-butylether, Di-iso-pentylether, Di-3-ethyldecylether, tert.-Butyl-n-octylether, iso-Pentyl-n-octylether und 2-Methyl-pentyl-n-octylether. Die als Handelsprodukte erhältlichen Verbindungen 1,3-Di-(2-ethyl-hexyl)-cyclohexan (Cetiol^{®} S) und Di-n-octylether (Cetiol^{®} OE) können bevorzugt sein.
- Esteröle. Unter Esterölen sind zu verstehen die Ester von C₆ - C₃₀ - Fettsäuren mit C₂ - C₃₀ - Fettalkoholen. Bevorzugt sind die Monoester der Fettsäuren mit Alkoholen mit 2 bis 24 C-Atomen. Beispiele für eingesetzte Fettsäurenanteile in den Estern sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen, bei der Oxidation von Aldehyden aus der Roelen'schen Oxosynthese oder der Dimerisierung von ungesättigten Fettsäuren anfallen. Beispiele für die Fettalkoholanteile in den Esterölen sind Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Erfindungsgemäß besonders bevorzugt sind Isopropylmyristat (Rilanit^{®} IPM), Isononansäure-C16-18-alkylester (Cetiol^{®} SN), 2-Ethylhexylpalmitat (Cegesoft^{®} 24), Stearinsäure-2-ethylhexylester (Cetiol^{®} 868), Cetyloleat, Glycerintricaprylat, Kokosfettalkohol-caprinatt-caprylat (Cetiol^{®} LC), n-Butylstearat, Oleylerucat (Cetiol^{®} J 600), Isopropylpalmitat (Rilanit^{®} IPP), Oleyl Oleate (Cetiol^{®}), Laurinsäurehexylester (Cetiol^{®} A), Di-n-butyladipat (Cetiol^{®} B), Myristylmyristat (Cetiol^{®} MM), Cetearyl Isononanoate (Cetiol^{®} SN), Ölsäuredecylester (Cetiol^{®} V).
- Dicarbonsäureester wie Di-n-butyladipat, Di-(2-ethylhexyl)-adipat, Di-(2-ethylhexyl)-succinat und Di-isotridecylacelaat sowie Diolester wie Ethylenglykol-dioleat, Ethylenglykol-di-isotridecanoat, Propylenglykol-di(2-ethylhexanoat), Propylenglykol-di-isostearat, Propylenglykol-di-pelargonat, Butandiol-di-isostearat, Neopentylglykoldicaprylat,
- symmetrische, unsymmetrische oder cyclische Ester der Kohlensäure mit Fettalkoholen, beispielsweise beschrieben in der DE-OS 197 56 454, Glycerincarbonat oder Dicaprylylcarbonat (Cetiol^{®} CC),
- Trifettsäureester von gesättigten und/oder ungesättigten linearen und/oder verzweigten Fettsäuren mit Glycerin,

Die Einsatzmenge der natürlichen und synthetischen kosmetischen Ölkörper in den erfindungsgemäß verwendeten Mitteln beträgt üblicherweise 0,1 - 30 Gew.%, bezogen auf das gesamte Mittel, bevorzugt 0,1 - 20 Gew.-%, und insbesondere 0,1 - 15 Gew.-%.

Erfindungsgemäß besonders bevorzugt sind Färbemittel, die mindestens einen Fettalkohol (D2) enthalten. Weiterhin haben sich Färbemittel als vorteilhaft erwiesen, die mindestens ein Esteröl enthalten. Ganz besonders bevorzugt können Färbemittel sein, die sowohl mindestens einen Fettalkohol (D2) als auch mindestens ein Esteröl enthalten.

Die Gesamtmenge an Öl- und Fettkomponenten in den erfindungsgemäßen Mitteln beträgt üblicherweise 0,5 - 75 Gew.-%, bezogen auf das gesamte Mittel. Mengen von 0,5 - 35 Gew.-% sind erfindungsgemäß bevorzugt.

Es hat sich als erfindungsgemäß besonders bevorzugt erwiesen, wenn die Färbemittel einen geringen Fettanteil (kleiner 20 Gew.-%) aufweisen. Darüber hinaus kann es erfindungsgemäß besonders bevorzugt sein, wenn die Färbemittel frei von Rohstoffen auf mineralischer und/oder tierischer Basis formuliert werden.

In einer weiteren Ausführungsform der vorliegenden Erfindung kann es bevorzugt sein, wenn die Färbemittel weiterhin mindestens ein Lösemittel enthalten. Während einige Färbegrundlagen des Standes der Technik empfindlich auf die Zugabe von Lösemitteln reagieren, zeichnet sich die erfindungsgemäße Cremegrundlage durch eine ausgezeichnete Lösemittelverträglichkeit aus. Erfindungsgemäß bevorzugte Lösemittel sind beispielweise Ethanol, Isopropanol, Ethylenglykol, Propylenglykol, Benzylalkohol, Methoxybutanol, Ethyldiglykol, Glycerin und Diethylenglykol. Besonders bevorzugte Lösemittel sind Propylenglykol, Benzylalkohol, Methoxybutanol und Ethyldiglykol.

Die erfindungsgemäßen Färbemittel enthalten Lösemittel bevorzugt in Mengen von 0,1 bis 15 Gew.-%, insbesondere von 2 bis 10 Gew.-%.

Die erfindungsgemäßen Färbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich. Ammoniak ist ein ganz besonders bevorzugtes Alkalisierungsmittel.

Auch Puffersysteme, wie beispielsweise Diammoniumhydrogenphoshat / Kaliumcarbonat, zur Einstellung eines bestimmten pH-Wertes können ohne Stabilitätsprobleme in die erfindungsgemäße Cremegrundlage eingearbeitet werden.

Die erfindungsgemäßen Färbemittel können weiterhin alle für solche Zubereitungen bekannten Wirk-, Zusatz- und Hilfsstoffe enthalten. In vielen Fällen enthalten die Färbemittel weiterhin mindestens ein anionisches, zwitterionisches, kationisches und/oder ampholytisches Tensid.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Ebenfalls insbesondere als Co-Tenside geeignet sind ampholytische Tenside. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Erfindungsgemäß werden als kationische Tenside insbesondere solche vom Typ der quartären Ammoniumverbindungen, der Esterquats und der Amidoamine eingesetzt.

Bevorzugte quaternäre Ammoniumverbindungen sind beispielsweise die unter den INCI-Bezeichnungen Quaternium-27 und Quaternium-83 bekannten Imidazolium-Verbindungen.

Bei Esterquats handelt es sich um bekannte Stoffe, die sowohl mindestens eine Esterfunktion als auch mindestens eine quartäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalze von Fettsäuren mit 1,2-Dihydroxypropyldialkylaminen. Solche Produkte werden beispielsweise unter den Warenzeichen Stepantex^{®}, Dehyquart^{®} und Armocare^{®} vertrieben. Die Produkte Armocare^{®} VGH-70, ein N,N-Bis(2-Palmitoyloxy-ethyl)dimethylammoniumchlorid, sowie Dehyquart^{®} F-75 und Dehyquart^{®} AU-35 sind Beispiele für solche Esterquats.

Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid^{®} S 18 im Handel erhältliche Stearamidopropyl-dimethylamin dar.

Weitere erfindungsgemäß verwendbare kationische Tenside stellen die quaternisierten Proteinhydrolysate dar.

Weitere erfindungsgemäß geeignete kationische Tenside sind die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA^{®}, Phospholipid PTC^{®} sowie Phospholipid SV^{®} vertrieben.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Ein Beispiel für ein als kationisches Tensid einsetzbares quaternäres Zuckerderivat stellt das Handelsprodukt Glucquat^{®}100 dar, gemäß INCI-Nomenklatur ein "Lauryl Methyl Gluceth-10 Hydroxypropyl Dimonium Chloride".

Bei den als Tensid eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so dass man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologehverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder-alkoholate als Katalysatoren verwendet werden. Die Verwendung von Produkten mit eingeengter Homologenverteilung kann bevorzugt sein. Es hat sich aber erfindungsgemäß als besonders vorteilhaft erwiesen, wenn die Färbemittel frei von anionischen, zwitterionischen und/oder ampholytischen Tensiden formuliert werden.

Ferner können die erfindungsgemäßen Färbemittel weitere Wirk-, Hilfs- und Zusatzstoffe, wie beispielsweise
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-tri-methylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert. Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- haarkonditionierende Verbindungen wie Phospholipide, beispielsweise Sojalecithin, Ei-Lecitin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, B₃, B₅, B₆, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,.
- Cholesterin,
- Konsistenzgeber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, N₂O, Dimethylether, CO₂ und Luft,
- Antioxidantien,
enthalten.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Ein zweiter Gegenstand der vorliegenden Erfindung sind Zweikomponentenmittel zur Färbung keratinischer Fasern, die aus einer ersten Zubereitung, enthaltend
(A) mindestens ein Farbstoffvorprodukt und/oder einen direktziehenden Farbstoff
(B) mindestens einen Fettsäureglycerylester und
(C) mindestens ein kationisches Tensid der Formel (I) 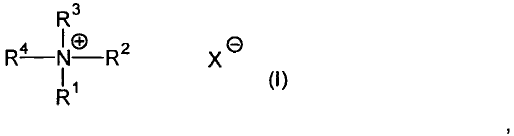 wobei die Reste R¹ bis R⁴ stehen unabhängig voneinander für eine C₁- bis C₄-Alkylkette oder eine C₁₀- bis C₃₀-Alkylkette, mit der Maßgabe, dass mindestens einer und höchstens zwei der Reste R¹ bis R⁴ für eine C₁₂- bis C₃₀-Alkylkette stehen, und X steht für ein physiologisch verträgliches Anion.
und einer zweiten Zubereitung, enthaltend mindestens ein Oxidationsmittel,
bestehen.

In einer bevorzugten Ausführungsform dieses erfindungsgemäßen Gegenstandes enthält die zweite Zubereitung mindestens ein anionisches oder mindestens ein kationisches Tensid. Hinsichtlich der erfindungsgemäß bevorzugten anionischen beziehungsweise kationischen Tenside sei an dieser Stelle auf die obigen Ausführungen verwiesen. Die erfindungsgemäßen Färbemittel dieser Ausführungsform zeichnen sich durch eine hervorragende Viskosität der resultierenden Anwendungszubereitung aus. Die Viskosität ist derart, dass die Mittel sich gut auf den Haaren verteilen lassen ohne anschließend abzutropfen.

Die erfindungsgemäßen Zweikomponentenmittel zeichnen sich durch eine gute Mischbarkeit, eine einfache Herstellbarkeit sowie eine außerordentliche Langzeitstabilität aus.

Erfolgt die Ausbildung der eigentlichen Färbung im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Bevorzugt wird jedoch ein chemisches Oxidationsmittel eingesetzt, besonders dann, wenn neben der Färbung ein Aufhelleffekt an menschlichem Haar gewünscht ist. Als Oxidationsmittel kommen Persulfate, Chlorite und insbesondere Wasserstoffperoxid oder dessen Anlagerungsprodukte an Harnstoff, Melamin sowie Natriumborat in Frage. Erfindungsgemäß kann aber das Oxidationsfärbemittel auch zusammen mit einem Katalysator auf das Haar aufgebracht werden, der die Oxidation der Farbstoffvorprodukte, z.B. durch Luftsauerstoff, aktiviert. Solche Katalysatoren sind z.B. Metallionen, Iodide, Chinone oder bestimmte Enzyme.

Geeignete Metallionen sind beispielsweise Zn²⁺, Cu²⁺, Fe²⁺, Fe³⁺, Mn²⁺, Mn⁴⁺, Li⁺, Mg²⁺, Ca²⁺ und Al³⁺. Besonders geeignet sind dabei Zn²⁺, Cu²⁺ und Mn²⁺. Die Metallionen können prinzipiell in der Form eines beliebigen, physiologisch verträglichen Salzes oder in Form einer Komplexverbindung eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Geeignete Enzyme sind z.B. Peroxidasen, die die Wirkung geringer Mengen an Wasserstoffperoxid deutlich verstärken können. Weiterhin sind solche Enzyme erfindungsgemäß geeignet, die mit Hilfe von Luftsauerstoff die Oxidationsfarbstoffvorprodukte direkt oxidieren, wie beispielsweise die Laccasen, oder in situ geringe Mengen Wasserstoffperoxid erzeugen und auf diese Weise die Oxidation der Farbstoff-vorprodukte biokatalytisch aktivieren. Besonders geeignete Katalysatoren für die Oxidation der Farbstoffvorläufer sind die sogenannten 2-Elektronen-Oxidoreduktasen in Kombination mit den dafür spezifischen Substraten, z.B.
- Pyranose-Oxidase und z.B. D-Glucose oder Galactose,
- Glucose-Oxidase und D-Glucose,
- Glycerin-Oxidase und Glycerin,
- Pyruvat-Oxidase und Benztraubensäure oder deren Salze,
- Alkohol-Oxidase und Alkohol (MeOH, EtOH),
- Lactat-Oxidase und Milchsäure und deren Salze,
- Tyrosinase-Oxidase und Tyrosin,
- Uricase und Harnsäure oder deren Salze,
- Cholinoxidase und Cholin,
- Aminosäure-Oxidase und Aminosäuren.

Das eigentliche oxidative Färbemittel wird zweckmäßigerweise unmittelbar vor der Anwendung durch Mischung der Zubereitung des Oxidationsmittels mit der Zubereitung, enthaltend die Farbstoffvorprodukte, hergestellt. Das dabei entstehende gebrauchsfertige Haarfärbepräparat sollte bevorzugt einen pH-Wert im Bereich von 5 bis 14, insbesondere von 7 bis 12, aufweisen. Besonders bevorzugt ist die Anwendung der Haarfärbemittel in einem schwach alkalischen Milieu. Die Anwendungstemperaturen können in einem Bereich zwischen 15 und 40 °C liegen. Nach einer Einwirkungszeit von 5 bis 45 Minuten wird das Haarfärbemittel durch Ausspülen von dem zu färbenden Haar entfernt. Das Nachwaschen mit einem Shampoo entfällt, wenn ein stark tensidhaltiger Träger, z.B. ein Färbeshampoo, verwendet wurde.

Insbesondere bei schwer färbbarem Haar kann die Zubereitung mit den Farbstoffvorprodukten aber auch ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Ein dritter Gegenstand der vorliegenden Erfindung ist daher ein Verfahren zur Färbung keratinischer Fasern, bei dem eines der erfindungsgemäßen Mittel gegebenenfalls mit einer Oxidationsmittelzubereitung vermischt wird, die Anwendungszubereitung auf die Fasern aufgetragen wird und nach einer Einwirkzeit wieder abgespült wird.

Besonders bevorzugt ist ein Verfahren zur Färbung keratinischer Fasern, bei dem ein erfindungsgemäßes Zweikomponentenmittel zu einer Anwendungszubereitung vermischt wird, diese auf die Fasern aufgetragen wird und nach einer Einwirkungszeit wieder abgespült wird.

Die nachfolgenden Beispiele sollen den Gegenstand der vorliegenden Erfindung verdeutlichen ohne ihn zu beschränken.

### Beispiele

Die in den folgenden Beispielen verwendeten Mengenangaben verstehen sich, sofern nichts anderes vermerkt ist, in Gewichtsprozent.

### Versuchsreihe A

Es wurden die folgenden Färbemittel hergestellt:

| Rohstoff | Färbecreme A1 | Färbecreme A2 | Färbecreme A3 |
|---|---|---|---|
| 1,2 Propylenglykol | 6,8 | 6,8 | 6,8 |
| Tetrasodium EDTA | 0,3 | 0,3 | 0,3 |
| Ascorbinsäure | 0,1 | 0,1 | 0,1 |
| Natriumsulfit | 0,3 | 0,3 | 0,3 |
| Parfüm | 0,5 | 0,5 | 0,5 |
| Ammoniak 25 % | 6,0 | 6,0 | 6,0 |
| Myristylalcohol | 0,9 | 0,9 | 0,9 |
| Cetearylalcohol | 7,1 | 7,1 | 7,1 |
| Hydrogenated Castor Oil | 0,7 | 0,7 | 0,7 |
| Glyceryl Stearate | 1,2 | 1,2 | 1,2 |
| Laureth-23 | 0,3 | 0,3 | 0,3 |
| Ceteareth-30 | 0,9 | 0,9 | 0,9 |
| Cetrimonium Cloride | 2,1 | 2,1 | 2,1 |
| Isopropylmyristate | 3,0 | 3,0 | 3,0 |
| p-Toluylendiamin-sulfat | 1,84 | --- | 5,2 |
| m-Aminophenol | 0,24 | 0,36 | 0,36 |
| Resorcin | 0,66 | --- | 1,76 |
| 2-Amino-4-(2-hydroxyethyl)aminoanisol-sulfat | 0,04 | --- | 0,18 |
| 1-(2-Hydroxyethyl)-4,5-diaminopyrazol | --- | 1,35 | --- |
| 4-Amino-3-methylphenol | --- | 0,14 | --- |
| 5-Amino-2-methylphenol | --- | 0,41 | --- |
| KOH, wäßrig | ad pH 10,3 | ad pH 10,6 | ad pH 10,0 |
| Wasser, entsalzt | ad 100 | ad 100 | ad 100 |
| Nuance der Ausfärbung | Hell-braun | Feuer-rot | Schwarz |

Jeweils 10ml der erfindungsgemäßen Färbecremes A1 bis A3 wurden mit 10ml der Oxidationsmittelzubereitung vermischt, die resultierenden Anwendungszubereitung mit einem Pinsel auf Fasern aus Büffelbauchhaar aufgetragen, dort 30 Minuten bei Raumtemperatur belassen und anschließend mit klarem Wasser gründlich abgespült. Die Fasern wurden mit einem Fön getrocknet und anschließend farbmetrisch vermessen. Dabei wurden die folgenden Färbungen erhalten:

| | L-Wert (Helligkeit) | a-Wert (Rot-/Grünanteil) | b-Wert (Gelb-/Blauanteil) |
|---|---|---|---|
| Färbecreme A1 | 21,3 | 2,1 | 2,9 |
| Färbecreme A2 | 33,1 | 40,6 | 22,4 |
| Färbecreme A3 | 18,2 | -0,2 | -2,8 |

### Versuchsreihe B

| Rohstoff | Färbecreme B1 | Färbecreme B2 | Färbecreme B3 |
|---|---|---|---|
| Glycerin | 5,0 | 5,0 | 5,0 |
| Tetrasodium EDTA | 0,3 | 0,3 | 0,3 |
| Ascorbinsäure | 0,1 | 0,1 | 0,1 |
| Natriumsulfit | 0,3 | 0,3 | 0,3 |
| Parfüm | 0,5 | 0,5 | 0,5 |
| Ammoniak 25 % | 5,6 | 5,6 | 5,6 |
| Ammoniumhydrogencarbonat | 2,0 | 2,0 | 2,0 |
| Myristylalcohol | 0,9 | 0,9 | 0,9 |
| Cetylalcohol | 6,6 | 6,6 | 6,6 |
| Hydrogenated Castor Oil | 0,7 | 0,7 | 0,7 |
| Glyceryl Stearate | 1,2 | 1,2 | 1,2 |
| Laureth-23 | 0,3 | 0,3 | 0,3 |
| Ceteareth-30 | 0,9 | 0,9 | 0,9 |
| Stearyltrimethyl Amonium Chloride | 3,6 | 3,6 | 3,6 |
| Isopropylpalmitat | 2,6 | 2,6 | 2,6 |
| Kieselsäure | 0,13 | 0,13 | 0,13 |
| p-Toluylendiamin-sulfat | 1,84 | -- | 5,2 |
| m-Aminophenol | 0,24 | 0,36 | 0,36 |
| Resorcin | 0,66 | -- | 1,76 |
| 2-Amino-4-(2-hydroxyethyl)aminoanisol-sulfat | 0,04 | -- | 0,18 |
| 1-(2-Hydroxyethyl)-4,5-diaminopyrazol | -- | 1,35 | -- |
| 4-Amino-3-methylphenol | -- | 0,14 | -- |
| 5-Amino-2-methylphenol | -- | 0,41 | -- |
| KOH, wäßrig | ad pH 10,3 | ad pH 10,6 | ad pH 10,0 |
| Wasser, entsalzt | ad 100 | ad 100 | ad 100 |
| Nuance der Ausfärbung | Hell-braun | Feuer-rot | Schwarz |

| Rohstoff | Färbecreme B4 | Färbecreme B5 | Färbecreme B6 |
|---|---|---|---|
| 1,2-Propylenglycol | 5,0 | 5,0 | 5,0 |
| Tetrasodium EDTA | 0,3 | 0,3 | 0,3 |
| Ascorbinsäure | 0,1 | 0,1 | 0,1 |
| Natriumsulfit | 0,3 | 0,3 | 0,3 |
| Parfüm | 0,5 | 0,5 | 0,5 |
| Ammoniak 25 % | 5,6 | 5,6 | 5,6 |
| Ammoniumhydrogencarbonat | 2,0 | 2,0 | 2,0 |
| Myristylalcohol | 0,9 | 0,9 | 0,9 |
| Cetylalcohol | 6,6 | 6,6 | 6,6 |
| Hydrogenated Castor Oil | 0,7 | 0,7 | 0,7 |
| Glyceryl Stearate | 1,2 | 1,2 | 1,2 |
| Laureth-23 | 0,3 | 0,3 | 0,3 |
| Ceteareth-30 | 0,9 | 0,9 | 0,9 |
| Stearyltrimethyl Amonium Chloride | 3,6 | 3,6 | 3,6 |
| Isopropylpalmitat | 2,6 | 2,6 | 2,6 |
| p-Toluylendiamin-sulfat | 1,84 | -- | 5,2 |
| m-Aminophenol | 0,24 | 0,36 | 0,36 |
| Resorcin | 0,66 | -- | 1,76 |
| 2-Amino-4-(2-hydroxyethyl)aminoanisol-sulfat | 0,04 | -- | 0,18 |
| 1-(2-Hydroxyethyl)-4,5-diaminopyrazol | -- | 1,35 | -- |
| 4-Amino-3-methylphenol | -- | 0,14 | -- |
| 5-Amino-2-methylphenol | -- | 0,41 | -- |
| KOH, wäßrig | ad pH 10,3 | ad pH 10,6 | ad pH 10,0 |
| Wasser, entsalzt | ad 100 | ad 100 | ad 100 |
| Nuance der Ausfärbung | Hell-braun | Feuer-rot | Schwarz |

| Rohstoff | Färbecreme B7 | Färbecreme B8 | Färbecreme B9 |
|---|---|---|---|
| Glycerin | 5,0 | 5,0 | 5,0 |
| Tetrasodium EDTA | 0,3 | 0,3 | 0,3 |
| Ascorbinsäure | 0,1 | 0,1 | 0,1 |
| Natriumsulfit | 0,3 | 0,3 | 0,3 |
| Parfüm | 0,5 | 0,5 | 0,5 |
| Ammoniak 25 % | 5,6 | 5,6 | 5,6 |
| Myristylalcohol | 0,9 | 0,9 | 0,9 |
| Cetylalcohol | 6,6 | 6,6 | 6,6 |
| Hydrogenated Castor Oil | 0,7 | 0,7 | 0,7 |
| Glyceryl Stearate | 1,2 | 1,2 | 1,2 |
| Laureth-23 | 0,3 | 0,3 | 0,3 |
| Ceteareth-30 | 0,9 | 0,9 | 0,9 |
| Stearyltrimethylamonium-Chloride | 3,6 | 3,6 | 3,6 |
| Isopropylpalmitat | 2,6 | 2,6 | 2,6 |
| Kieselsäure | 0,13 | 0,13 | 0,13 |
| p-Toluylendiamin-sulfat | 1,84 | -- | 5,2 |
| m-Aminophenol | 0,24 | 0,36 | 0,36 |
| Resorcin | 0,66 | -- | 1,76 |
| 2-Amino-4-(2-hydroxyethyl)aminoanisol-sulfat | 0,04 | -- | 0,18 |
| 1-(2-Hydroxyethyl)-4,5-diaminopyrazol | -- | 1,35 | -- |
| 4-Amino-3-methylphenol | -- | 0,14 | -- |
| 5-Amino-2-methylphenol | -- | 0,41 | -- |
| KOH, wäßrig | ad pH 10,3 | ad pH 10,6 | ad pH 10,0 |
| Wasser, entsalzt | ad 100 | ad 100 | ad 100 |
| Nuance der Ausfärbung | Hell-braun | Feuer-rot | Schwarz |

Jeweils 10ml der erfindungsgemäßen Färbecremes B1 bis B9 wurden mit 10ml der Oxidationsmittelzubereitung vermischt, die resultierenden Anwendungszubereitung mit einem Pinsel auf Fasern aus Büffelbauchhaar aufgetragen, dort 30 Minuten bei Raumtemperatur belassen und anschließend mit klarem Wasser gründlich abgespült. Die Fasern wurden mit einem Fön getrocknet und anschließend farbmetrisch vermessen. Dabei wurden die folgenden Färbungen erhalten:

| | L-Wert (Helligkeit) | a-Wert (Rot-/Grünanteil) | b-Wert (Gelb-/Blauanteil) |
|---|---|---|---|
| Färbecreme B1 | 21,3 | 2,1 | 2,9 |
| Färbecreme B2 | 33,1 | 40,6 | 22,4 |
| Färbecreme B3 | 18,2 | -0,2 | -2,8 |
| Färbecreme B4 | 21,3 | 2,1 | 2,9 |
| Färbecreme B5 | 33,1 | 40,6 | 22,4 |
| Färbecreme B6 | 18,2 | -0,2 | -2,8 |
| Färbecreme B7 | 21,3 | 2,1 | 2,9 |
| Färbecreme B8 | 33,1 | 40,6 | 22,4 |
| Färbecreme B9 | 18,2 | -0,2 | -2,8 |

Der Einsatz von Ammoniumhydrogencarbonat und/oder Kieselsäure wirkt sich auf das Farbergebnis (die Färbung) nicht aus, führt aber zu verbesserten Farbleistungen der Färbecremes.

## Patentansprüche

1. Mittel zur Färbung keratinischer Fasern, enthaltend
(A) mindestens ein Farbstoffvorprodukt
(B) mindestens einen Fettsäureglycerylester und
(C) mindestens ein kationisches Tensid der Formel (I) 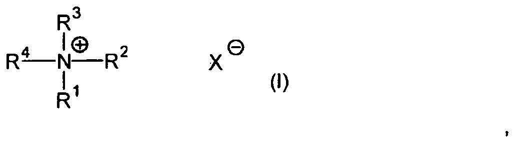
wobei die Reste R¹ bis R⁴ stehen unabhängig voneinander für eine C₁- bis C₄-Alkylkette oder eine C₁₀- bis C₃₀-Alkylkette, mit der Maßgabe, dass mindestens einer und höchstens zwei der Reste R¹ bis R⁴ für eine C₁₂- bis C₃₀-Alkylkette stehen, und X steht für ein physiologisch verträgliches Anion,
wobei das Mittel die Fettsäureglycerylester in Mengen von 0,1 bis 10 Gew.-% enthält und das Mittel weiterhin mindestens ein vollständig oder partiell hydriertes Pflanzenöl enthält.

2. Mittel nach Anspruch 1, **dadurch gekennzeichnet, dass** als Farbstoffvorprodukt mindestens eine Entwickler- oder Kupplerkomponente enthalten ist.

3. Mittel nach einem der Ansprüche 1 oder 2, **dadurch gekennzeichnet, dass** als Farbstoffvorprodukt mindestens ein Indol- und/oder Indolinderivat enthalten ist.

4. Mittel nach einem der Ansprüche 1 bis 3, **dadurch gekennzeichnet, dass** es weiterhin mindestens einen direktziehenden Farbstoff enthält.

5. Mittel nach einem der Ansprüche 1 bis 4, **dadurch gekennzeichnet, dass** es als Fettsäureglycerylester Ölsäuremonoglyceride und/oder Stearinsäuremonoglyceride enthält.

6. Mittel nach einem der Ansprüche 1 bis 5, **dadurch gekennzeichnet, dass** die Verbindung der Formel (I) ausgewählt ist aus Alkyltrimethylammoniumchloriden, Dialkyldimethylammoniumchloriden, Alkyltrimethylammoniumbromiden und Dialkyldimethylammoniumbromiden.

7. Mittel nach einem der Ansprüche 1 bis 6, **dadurch gekennzeichnet, dass** die Verbindung der Formel (I) ausgewählt ist aus Dicaprindimethylammoniumchlorid, Lauryltrimethylammoniumchlorid, Cetyltrimethylammoniumchlorid, Stearyltrimethylammoniumchlorid, Distearyldimethylammoniumchlorid, Dicetyldimethylammoniumchlorid, Dilauryldimethylammoniumchlorid, Behenyltrimethylammoniumchlorid und Behenyltrimethylammoniummethosulfat.

8. Mittel nach Anspruch 1, **dadurch gekennzeichnet, dass** das vollständig oder partiell hydrierte Pflanzenöl ausgewählt ist aus Sojabohnenöl, Kokosnussöl, Haselnussöl, Palmkernöl, Sonnenblumenöl, Leinsamenöl, Erdnussöl, Rizinusöl oder Rapsöl.

9. Mittel nach einem der Ansprüche 1 bis 8, **dadurch gekennzeichnet, dass** es weiterhin Ammoniumhydrogencarbonat enthält.

10. Mittel nach Anspruch 9, **dadurch gekennzeichnet, dass** es - bezogen auf das Gewicht des Mittels - 0,1 bis 10 Gew.-%, vorzugsweise 0,25 bis 8 Gew.-%, besonders bevorzugt 0,5 bis 5 Gew.-% und insbesondere 1 bis 3 Gew.-% Ammoniumhydrogencarbonat, enthält.

11. Mittel nach einem der Ansprüche 1 bis 10, **dadurch gekennzeichnet, dass** es weiterhin Kieselsäure enthält.

12. Mittel nach Anspruch 11, **dadurch gekennzeichnet, dass** es - bezogen auf das Gewicht des Mittels - 0,05 bis 5 Gew.-%, vorzugsweise 0,1 bis 3 Gew.-%, besonders bevorzugt 0,15 bis 2 Gew.-% und insbesondere 0,2 bis 1 Gew.-% Kieselsäure, enthält.

13. Zweikomponentenmittel zur Färbung keratinischer Fasern, **dadurch gekennzeichnet, dass** es eine erste Zubereitung nach einem der Ansprüche 1 bis 12 enthält und eine zweite Zubereitung enthaltend mindestens ein Oxidationsmittel.

14. Zweikomponentenmittel nach Anspruch 13, **dadurch gekennzeichnet, dass** die zweite Zubereitung mindestens ein anionisches oder kationisches Tensid enthält.

15. Verfahren zur Färbung keratinischer Fasern, **dadurch gekennzeichnet, dass** ein Zweikomponentenmittel nach einem der Ansprüche 13 oder 14 zu einer Anwendungszubereitung vermischt wird, diese auf die Fasern aufgetragen wird und nach einer Einwirkungszeit wieder abgespült wird.

## Claims

1. Agent for dyeing keratinic fibers, containing
(A) at least one dye intermediate product
(B) at least one fatty acid glyceryl ester and
(C) at least one cationic surfactant of formula (I) 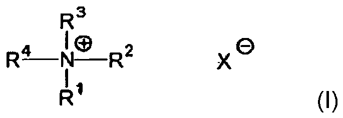
wherein the radicals R¹ to R⁴ independently stand for a C₁ to C₄ alkyl chain or a C₁₀ to C₃₀ alkyl chain, with the condition that at least one and a maximum of two of the radicals R¹ to R⁴ stand for a C₁₂ to C₃₀ alkyl chain, and X stands for a physiologically acceptable anion, wherein the agent contains the fatty acid glyceryl esters in quantities of 0.1 to 10% by weight, and the agent further contains at least one completely or partially hydrogenated vegetable oil.

2. Agent according to Claim 1, **characterized in that** at least one developer component or coupler component is contained as the dye intermediate product.

3. Agent according to one of Claims 1 or 2, **characterized in that** at least one indol derivative and/or indoline derivative is contained as the dye intermediate product.

4. Agent according to one of Claims 1 to 3, **characterized in that** the agent also contains at least one substantive dye.

5. Agent according to one of Claims 1 to 4, **characterized in that** the agent contains oleic acid monoglycerides and/or stearic acid monoglycerides as fatty acid glyceryl ester.

6. Agent according to one of Claims 1 to 5, **characterized in that** the compound of formula (I) is selected from alkyltrimethylammonium chlorides, dialkyldimethylammonium chlorides, alkyltrimethylammonium bromides, and dialkyldimethylammonium bromides.

7. Agent according to one of Claims 1 to 6, **characterized in that** the compound of formula (I) is selected from dicapryldimethylammonium chloride, lauryltrimethylammonium chloride, cetyltrimethylammonium chloride, stearyltrimethylammonium chloride, distearyldimethylammonium chloride, dicetyldimethylammonium chloride, dilauryldimethylammonium chloride, behenyltrimethylammonium chloride, and behenyltrimethylammonium methosulfate.

8. Agent according to Claim 1, **characterized in that** the completely or partially hydrogenated vegetable oil is selected from soybean oil, coconut oil, hazelnut oil, palm kernel oil, sunflower oil, flaxseed oil, peanut oil, castor oil, or rapeseed oil.

9. Agent according to one of Claims 1 to 8, **characterized in that** the agent also contains ammonium hydrogen carbonate.

10. Agent according to Claim 9, **characterized in that** the agent contains 0.1 to 10% by weight, preferably 0.25 to 8% by weight, particularly preferably 0.5 to 5% by weight, and in particular 1 to 3% by weight ammonium hydrogen carbonate, based on the weight of the agent.

11. Agent according to one of Claims 1 to 10, **characterized in that** agent also contains silicic acid.

12. Agent according to Claim 11, **characterized in that** the agent contains 0.05 to 5% by weight, preferably 0.1 to 3% by weight, particularly preferably 0.15 to 2% by weight, and in particular 0.2 to 1% by weight silicic acid, based on the weight of the agent.

13. Two-component agent for dyeing keratinic fibers, **characterized in that** the two-component agent contains a first preparation according to one of Claims 1 to 12 and a second preparation containing at least one oxidizing agent.

14. Two-component agent according to Claim 13, **characterized in that** the second preparation contains at least one anionic or cationic surfactant.

15. Method for dyeing keratinic fibers, **characterized in that** a two-component agent according to one of Claims 13 or 14 is mixed with an application preparation, said mixture is applied to the fibers, and the mixture is rinsed off after an exposure period.

## Revendications

1. Agent de teinture de fibres kératiniques, ledit agent comprenant
(A) au moins un précurseur de colorant
(B) au moins un ester glycérylique d'acide gras et
(C) au moins un tensioactif cationique de formule la (I) 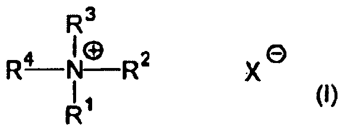
où les radicaux R¹ à R⁴ représentent chacun indépendamment l'un de l'autre une chaîne alkyle en C₁-C₄ ou une chaîne alkyle en C₁₀-C₃₀, avec la condition qu'au moins un et au plus deux des radicaux R₁ à R⁴ représentent une chaîne alkyle en C₁₂-C₃₀ et X représente un anion physiologiquement acceptable,
l'agent contenant les esters glycéryliques d'acide gras dans des quantités allant de 0,1 à 10% en poids et l'agent contenant en outre au moins une huile végétale totalement ou partiellement hydrogénée.

2. Agent selon la revendication 1, **caractérisé en ce que** le précurseur de colorant contenu est au moins un composant de développement ou de couplage.

3. Agent selon l'une des revendications 1 ou 2, **caractérisé en ce que** le précurseur de colorant contenu est au moins un dérivé de l'indole et/ou de l'indoline.

4. Agent selon l'une des revendications 1 à 3, **caractérisé en ce qu'**il contient en outre au moins un colorant direct.

5. Agent selon l'une des revendications 1 à 4, **caractérisé en ce qu'**il contient comme esters glycéryliques d'acide gras des monoglycérides d'acide oléique et/ou des monoglycérides d'acide stéarique.

6. Agent selon l'une des revendications 1 à 5, **caractérisé en ce que** le composé de la formule (I) est choisi parmi le chlorure d'alkyltriméthylammonium, le chlorure de dialkyldiméthylammonium, le bromure d'alkyltriméthylammonium et le bromure de dialkyldiméthylammonium.

7. Agent selon l'une des revendications 1 à 6, **caractérisé en ce que** le composé de la formule (I) est choisi parmi le chlorure de dicaprindiméthylammonium, le chlorure de lauryltriméthylammonium, le chlorure de cétyltriméthylammonium, le chlorure de stéaryltriméthylammonium, le chlorure de distéaryldiméthylammonium, le chlorure dicétyldiméthylammonium, le chlorure de dilauryldiméthylammonium, le chlorure de béhényltriméthylammonium et le méthosulfate de béhényltriméthylammonium.

8. Agent selon la revendication 1, **caractérisé en ce que** l'huile végétale totalement ou partiellement hydrogénée est choisie parmi l'huile de soja, l'huile de noix de coco, l'huile de noisette, l'huile de palmiste, l'huile de tournesol, l'huile de lin, l'huile d'arachide, l'huile de ricin ou l'huile de colza.

9. Agent selon l'une des revendications 1 à 8, **caractérisé en ce qu'**il contient en outre du hydrogénocarbonate d'ammonium.

10. Agent selon la revendication 9, **caractérisé en ce qu'**il contient, par rapport au poids de l'agent, 0,1 à 10% en poids, de préférence de 0,25 à 8% en poids, de façon particulièrement préférée de 0,5 à 5% en poids et notamment de 1 à 3% en poids d'hydrogénocarbonate d'ammonium.

11. Agent selon l'une des revendications 1 à 10, **caractérisé en ce qu'**il contient en outre de l'acide silique.

12. Agent selon la revendication 11, **caractérisé en ce qu'**il contient, par rapport au poids de l'agent, 0,05 à 5% en poids, de préférence 0,1 à 3% en poids, de manière particulièrement préférée 0,15 à 2% en poids et notamment 0,2 à 1% en poids d'acide silique.

13. Agent à deux composants pour la teinture de fibres kératiniques, **caractérisé en ce qu'**il contient une première préparation selon l'une des revendications 1 à 12 et une seconde préparation contenant au moins un agent oxydant.

14. Agent à deux composants selon la revendication 13, **caractérisé en ce que** la seconde composition contient au moins un tensioactif anionique ou cationique.

15. Procédé de teinture de fibres kératiniques, **caractérisé en ce qu'**un agent à deux composants selon l'une des revendications 13 ou 14 est mélangé à une préparation d'application et **en ce que** celle-ci est appliquée sur les fibres et est à nouveau lavée au bout d'un certain temps d'imprégnation.
